# EUROPÄISCHE PATENTSCHRIFT

(11) **EP 1 025 123 B1**
(45) Veröffentlichungstag und Bekanntmachung des Hinweises auf die Patenterteilung: **16.12.2009**
(21) Anmeldenummer: 98955520.6
(22) Anmeldetag: 23.10.1998
(51) Int. Cl.: C07K 14/52, C12P 21/02, A61K 38/20, A61K 38/19

(54) **VERFAHREN ZUR HERSTELLUNG VON HUMANEIGENBLUTZYTOKINEN**
METHOD FOR THE PRODUCTION OF HUMAN BLOOD CYTOKINES
PROCEDE POUR LA PRODUCTION DE CYTOKINES DE SANG AUTOLOGUE HUMAIN

(30) Priorität: 23.10.1997 DE 19746865; 15.05.1998 DE 19821843
(43) Veröffentlichungstag der Anmeldung: 09.08.2000
(73) Patentinhaber: Klehr, Nikolaus W., 81545 München (DE)
(72) Erfinder: Klehr, Nikolaus W., 81545 München (DE)
(74) Vertreter: Grättinger Möhring von Poschinger Patentanwälte Partnerschaft
(86) Internationale Anmeldenummer: PCT/EP1998/006757
(87) Internationale Veröffentlichungsnummer: WO 1999/021887

(56) Entgegenhaltungen:
- WO-A-95/07105
- WO-A-97/05239
- DE-A- 3 923 848
- NICOS A NICOLA (ED.): "Guidebook to cytokines and their receptors" 1994 , SAMBROOK AND TOOZE PUBLICATIONS , OXFORD GB XP002099612 siehe Seite 103 - Seite 104 siehe Seite 118 - Seite 119

## Beschreibung

Die Erfindung betrifft ein Verfahren zur Herstellung von Humaneigenblutzytokinen.

Es ist bekannt, daß eine Vielzahl von Menschen im Laufe ihres Lebens an Tumoren erkranken und daran sterben. Bei einem Tumor handelt es sich um ursprünglich körpereigene Zellen, die durch die verschiedensten Einflüsse zu Tumorzellen entarten können. Solche Zellen weisen ein abnormales Zellwachstum auf, das letztendlich auf gesunde Zellen destruktiv wirkt.

Bei der Behandlung von Tumorerkrankungen steht es im Vordergrund, die Tumorzellen operativ zu entfernen. Eine solche Operation ist jedoch für den - bereits durch den Tumor geschwächten - Patienten sehr belastend. Ferner ist nicht immer sichergestellt, daß bei der Operation tatsächlich alle Tumorzellen entfernt werden. Nicht entfernte Tumorzellen können jedoch weiter wachsen und somit zu einem erneuten Ausbruch der Tumorerkrankung führen. Weiterhin werden durch die operative Entfernung des Primärtumors die Metastasen (Tochtergeschwülste) nicht erfaßt, die ebenfalls zu einer Tumorerkrankung führen können.

Nach der operativen Entfernung von Tumorzellen wird der Patient häufig einer Chemotherapie und/oder einer Strahlentherapie unterzogen, um eventuell noch im Körper vorhandene Tumorzellen abzutöten. Beide Therapien werden jedoch von massiven Nebenwirkungen begleitet, wie starkes Erbrechen, Gewichtsverlust und Haarausfall. Des weiteren werden durch Chemotherapeutika und Strahlen, die beide selbst Krebserzeugend sind, neue Tumorzellen erzeugt, die zur Entstehung eines neuen Tumors beitragen können.

Da die vorstehenden Therapien zur Tumorbehandlung nicht befriedigend sind, wird verschiedentlich versucht, das körpereigene Immunsystem des Patienten zur Behandlung von Tumoren auszunutzen. Dabei soll das Immunsystem des Patienten dazu gebracht werden, Tumorzellen als körperfremd zu erkennen und zu vernichten. Dies hat sich jedoch als äußerst schwierig herausgestellt, da sich die Tumorzellen maskieren und somit für das körpereigene Immunsystem nicht erkennbar sind.

Ein Ansatz zur Überwindung dieses Problems ist in der DE-A-39 23 848 aufgezeigt. Diese Patentanmeldung betrifft ein Verfahren zur Immunpotenzierung von weißen Blutzellen, bei dem Tumorzellen durch einen chirurgischen Eingriff gewonnen und dann mit autologen weißen Blutzellen überschichtet werden. Dabei werden immunpotenzierte weiße Blutzellen erhalten, die dem Patienten reinjeziert werden. Dies führt letztendlich zur Zerstörung des Tumors durch die immunpotenzierten weißen Blutzellen. Allerdings weist dieses Verfahren den Nachteil auf, daß ein chirurgischer Eingriff notwendig ist, der den Patienten stark belastet.

Der vorliegenden Erfindung liegt somit die Aufgabe zugrunde, ein Verfahren zur Herstellung eines Mittels zu schaffen, das nicht die Nachteile des Standes der Technik aufweist.

Erfindungsgemäß wird dies durch ein Verfahren zur Herstellung von Humaneigenblutzytokinen erreicht, das die folgenden Schritte umfaßt:
a) Gewinnen einer Zusammensetzung von weißen Blutzellen, Plasma und einem Antigen aus dem Vollblut eines Spenders, wobei diese von den Erythrozyten abgetrennt werden,
b) Aufteilen der Zusammensetzung in ein erstes und ein zweites Aliquot,
c) Demaskieren der im ersten Aliquot vorliegenden Zellen und
d) Zusammengeben des in Schritt c) erhaltenen ersten Aliquots zum zweiten Aliquot und nachfolgende Inkubation.

Die Erfindung beruht auf folgender Erkenntnis des Erfinders: Im Blut eines Tumorpatienten liegen - neben den üblichen weißen Blutzellen - Tumorzellen und Tumorzellmaterial-enthaltende weiße Blutzellen vor. Die beiden letzteren sind aber so "maskiert", daß sie von den weißen Blutzellen nicht als fremd erkannt werden und daher auch keine Immunantwort gegen den Tumor induziert wird. Diese Maskierung der Tumorzellen ist darauf zurückzuführen, daß sie Oberflächenmarker, wie CD44V aufweisen, die von den weißen Blutzellen nicht als fremd erkannt werden. Führt man jedoch das erfindungsgemäße Verfahren durch, dann werden die weißen Blutzellen dazu gebracht, vermehrt Zytokine zu produzieren. Nach Schritt d) des erfindungsgemäßen Verfahrens wird somit ein Produkt erhalten, das autologe Zytokine in einem effizient erhöhte Gehalt aufweist. Reinjeziert man daher dieses Produkt dem Spender, aus dem es gewonnen wurde, dann kommt es zu einer spezifischen, gegen den Tumor gerichteten Immunantwort über die Zytokinproduktion und somit zu einer Heilung der Tumorerkrankung.

Diese Erkenntnis trifft nicht nur für Tumoren und Tumorzellen zu, sondern auch für Viren, Bakterien, Prionen und andere Antigene, die zum Ausbruch einer damit assoziierten Krankheit führen können und im Blut von Menschen vorkommen.

Der Ausdruck "Humaneigenblutzytokine" umfaßt Zytokine jeglicher Art, die in menschlichem Blut vorkommen, wobei der Begriff "...eigen..." darauf hinweist, daß die Zytokine autologe Zytokine sind, d.h. vom gleichen Menschen stammen.

Zytokine sind Botenstoffe des Immunsystems, die die Proliferation und Differenzierung von Blutzellen regulieren. Sie werden in folgende Unterklassen-eingeteilt: Interleukine, Interferone, Kolonie-stimulierende Faktoren, Lymphokine und Wachstumsfaktoren. Für das erfindungsgemäße Verfahren sind die Zytokine vorzugsweise ausgewählt aus Tumornekrosefaktor-a, Interferon-g und löslichem Interleukin-2-Rezeptor und Gemische von zwei bis allen der Interleukine.

Im erfindungsgemäßen Verfahren werden Humaneigeblutzytokine hergestellt. Diese Herstellung umfaßt auch die Konzentrationserhöhung der Zytokine, wobei die Zytokinkonzentration vor und nach Durchführung des erfindungsgemäßen Verfahrens bestimmt werden kann. Dabei ist es ausreichend, daß die Konzentration von mindestens zwei der Zytokine im Vergleich zu unbehandeltem Blut durch das erfindungsgemäße Verfahren erhöht ist.

In Schritt a) wird eine Zusammensetzung von weißen Blutzellen, Plasma und einem Antigen aus dem Vollblut eines Spenders gewonnen, wobei diese von den Erythrozyten abgetrennt werden. Der Ausdruck "weiße Blutzellen" umfaßt im Blut vorkommende kernhaltigen Zellen jeglicher Art, insbesondere Leukozyten, z.B. Lymphozyten, Monozyten und neutrophile, eosinophile und basophile Granulozyten. Die vorstehende Zusammensetzung kann auf jede beliebige Art gewonnen werden, mit der weiße Blutzellen, Plasma und Antigene, wie Tumorzellen, von den Erythrozyten getrennt werden. Als günstig hat es sich für die Gewinnung der Zusammensetzung gezeigt, wie folgt vorzugehen. Zuerst wird dem Patienten in üblicher Weise Vollblut entnommen, z.B. aus der Ellenbeugenvene, das dann mit Heparin versetzt wird. Das Vollblut weist dabei die Antigen-wirkenden Zllen auf. Es wird so viel Vollblut dem Patienten entnommen, um zum einen die unproblematische Durchführung des Verfahrens zu gewährleisten und zum anderen den Patienten nicht übermäßig zu belasten. Als geeignetes Volumen haben sich dabei ca. 50 ml Vollblut erwiesen. Eine Weiterverarbeitung des heparinisierten Vollblutes erfolgt günstigerweise innerhalb 96 Stunden nach der Blutentnahme, wobei eine besondere Kühlung des heparinisierten Blutes in dieser Zeitspanne nicht notwendig ist, d.h. es kann bei Raumtemperatur bis zur Weiterverarbeitung gelagert werden. Dies stellt im Hinblick auf die Wirtschaftlichkeit des erfindungsgemäßen Verfahrens einen großen Vorteil dar. Das heparinisierte Vollblut wird dann in ein Röhrchen , z.B. der Entnahmespritze, senkrecht stehen gelassen, wobei die Schwerkraft auf das Blut einwirkt. Durch das Einwirken der Schwerkraft kommt es zur Ausbildung von drei Schichten. Die unterste Schicht sind die Erythrozyten, darüber befinden sich die weißen Blutzellen (Interphase), gefolgt vom Plasma, wobei entweder in der Interphase oder im Plasma oder in beiden sich das Antigen befindet. Die Abtrennung der Interphase und des Plasmas von den Erythrozyten kann auf jede beliebige Art erfolgen. Beispielsweise wird an der Spritze, mit der das Blut entnommen wurde, ein Schlauch angebracht. Ferner werden vier Röhrchen, z.B. ein 50 ml-Röhrchen, ein 15 ml-Röhrchen und zwei Röhrchen mit je 10 ml Inhalt, bereitgestellt. In das erste 10 ml-Röhrchen wird ein Teil des Plasmas, z.B. 2 ml, überführt, in dem der Zytokingehalt (= Zytokin-Ausgangswert) bestimmt werden kann. Auch in das zweite 10 mlRöhrchen wird ein Teil des Plasmas, z.B. 2 ml, eingeführt, in dem die infektionsserologische Parameter, wie anti-HCV, anti-HIV-1-2 und HbsAg, bestimmt werden können. Anschließend wird der Schlauch in das Röhrchen mit 50 ml Inhalt eingelegt, und es erfolgte die Überführung des restlichen Plasmas bis die Erythrozytenschicht (sichtbare rötliche Verfärbung des Schlauches) erreicht wird. Unter Beibehaltung des Drucks auf den Spritzenstempel und weitere Überführung des Blutes in das Röhrchen nimmt die Fließfähigkeit des Blutes ab. Der Druck auf den Spritzenstempel wirde solange fortgesetzt, bis die Fließfähigkeit wieder den Vorzustand (Einzeitropfenbiläung) erreicht hat. Danach war die Überführung des Spritzeninhalts in das Röhrchen beendet. Diese Schritte können bei Raumtemperatur durchgeführt werden.

Die vorstehend erwähnten Bestimmungsverfahren für Zytokine und infektionsserologische Parameter sowie hierzu notwendige Materialien und Geräte sind dem Fachmann bekannt.

In Schritt b) des erfindungsgemäßen Verfahrens wird die in Schritt a) gewonnene Zusammensetzung in zwei Aliquote, nämlich in ein erstes und ein zweites Aliquot, aufgeteilt. Vorzugsweise erfolgt das Aufteilen in Schritt b) so, daß das erste Aliquot 10 bis 40 Vol.-%, vorzugsweise 20 bis 30 Vol.%, insbesondere ca. 25 Vol.-%, des gesamten Volumens der Zusammensetzung beträgt. Dieses Aufteilen kann z.B. dadurch erfolgen, daß das erste Aliquot, z.B. mit einer Spritze, entnommen und in ein anderes Gefäß transferiert wird.

In Schritt c) des erfindungsgemäßen Verfahrens werden die im ersten Aliquot enthaltenen Zellen demaskiert. Diese Zellen weisen die weißen Blutzellen, die weißen Blutzellen, die bereits die Antigene oder Teile davon enthalten, und die Antigene selbst auf. Der Ausdruck "Demaskieren" bedeutet, daß die Zellen diejenigen Eigenschaften verlieren, die sie für das körpereigene Immunsystem unkenntlich machen. Das Demaskieren umfaßt die Entfernung der Tarnung der Zellen, insbesondere der Tumorzellen, und/oder die Membranreduktion der Zellen. Das Demaskieren kann spontan erfolgen oder induziert werden. Günstig ist es dabei, wenn die Induktion der Demaskierung physikalisch erfolgt. Dies bedeutet, daß die Zellen physikalisch, z.B. mechanisch, so beeinflußt werden, daß sie demaskiert werden. Als besonders brauchbare Maßnahme für das physikalische Induzieren hat sich das Zentrifugieren, z.B. bei 1800 Umdrehungen pro Minute während 10 Minuten erwiesen. Nach der Zentrifugation kann der Überstand abgenommen werden, z.B. durch Dekantation, und zum zweiten Aliquot zugegeben werden. Der Rückstand (demaskierte Zellen) wird in einem geeigneten wäßrigen Medium, z.B. Ringerphosphatlösung, aufgenommen und für das weitere Verarbeiten, d.h. Schritt d) des erfindungsgemäßen Verfahrens, verwendet.

In Schritt d) werden das erste Aliquot, das die demaskierten Zellen enthält, oder das diesem ersten Aliquot entsprechende oben genannte Medium, das ebenfalls die demaskierten Zellen enthält, mit dem zweiten Aliquot zusammengegeben, vorzugsweise in einer Zellkulturflasche. Anschließend wird eine Inkubation durchgeführt, vorzugsweise bei 37 ± 2 °C während 48 ± 5 bis 72 ± 5 Stunden. Das nach Schritt d) erhaltene Produkt kann in Ampullen in üblicher Weise konfektioniert werden und/oder bei ca. - 16 °C oder weniger während 12 Stunden bis 14 Tagen gelagert werden.

Das erfindungsgemäße Verfahren wird vorzugsweise unter sterilen Bedingungen durchgeführt. Beispielsweise werden die verwendeten Geräte und Verbrauchsmaterialien in üblicher Weise, z.B. mit einem Sterilisationsmittel, wie einen Alkohol oder eine alkoholische Lösung, behandelt und/oder unter Reinluftbedingungen gearbeitet.

Das erfindungsgemäße Verfahren weist eine Reihe von Vorteilen auf: Es kann ein wirksames Mittel gegen die durch die weiter oben genannten Antigene verursachten Erkrankungen, insbesondere Tumorerkrankungen, in schneller, preisgünstiger Weise ohne großen Apparateaufwand schaffen. Dieses kann als naürliches, biologisches Medikament angesehen werden, da es nur natürlich vorkommende Substanzen enthält und ohne Einsatz von Fremdstoffen hergestellt wurde. Im Vergleich zu vielen anderen Mitteln, wie Antitumormittel, ist seine Verwendung und Herstellung gefahrlos möglich. Das durch das erfindungsgemäße Verfahren hergestellt Mittel ist nebenwirkungsfrei und es zeigt eine optimale, direkte therapeutische Wirkung. Ferner hält es mit der Tumorevolution schritt, da es dem Patienten in verschiedenen Zeitabständen verabreicht werden kann und es sich dabei jeweils dem aktuellen Evolutionsstadium des Tumors anpaßt.

Das durch das erfindungsgemäße Verfahren hergestellte Zytokin-haltige Produkt ist daher zur Behandlung von durch die Antigene verursachten Erkrankungen, insbesondere Tumorerkrankungen, bestens geeignet.

Wie vorstehend dargelegt wurde, kann vor und nach der Durchführung des erfindungsgemäßen Verfahrens jeweils die Konzentration der Zytokine bestimmt werden. Ist die Konzentration von mindestens zwei Zytokinen nach der Durchführung der erfindungsgemäßen Verfahrens erhöht, dann ist dies ein Hinweis darauf, daß zum einen die Durchführung des erfindungsgemäßen Verfahrens erfolgreich war und zum anderen, daß die entsprechenden Antigene im Blut vorliegen. Somit ist das erfindungsgemäße Verfahren auch bestens zur Diagnose der durch die Antigene verursachten Erkrankungen einsetzbar.

Das folgende Beispiel erläutert die Erfindung:

### Beispiel: Herstellung von Humaneigenblutzytokinen

Heparinisiertes Vollblut wurde einem Tumorpatienten wie folgt entnommen: Die zur Blutentnahme vorgesehene Stelle an der Ellenbeugenvene wurde mit einer alkoholischen Lösung desinfiziert. Anschließend wurde eine Ampulle, die Heparin enthielt, aufgebrochen und das Heparin über eine sterile Kanüle in eine Entnahnamespritze aspiriert. Nachdem durch Anlegen eines Stauschlauches am Oberarm die Ellenbeugenvene gestaut wurde, wurde sie punktiert und in üblicher Weise der Erfolg der Punktion kontrolliert. Der Blutstau wurde durch Lösen der Stauschlauches beseitigt und 50 ml Vollblut wurde in die bereits Heparin enthaltende Spritze aspiriert. Die Spritze wurde aus der Vene heraus gezogen. Das so gewonnene heparinisierte Vollblut wurde innerhalb von 96 Stunden weiterverarbeitet, wobei während der Lagerung bis zur Weiterverarbeitung die Spritze bei Raumtemperatur aufbewahrt wurde.

Die Spritze wurde dann vertikal mit der Spritze nach oben in einen Edelstahlbehälter gestellt. Hier verblieb die Spritze bei Raumtemperatur, bis durch die Schwerkraftsedimentation der Zellen die Interphase eintrat. Diese ist dadurch erkennbar, daß sich eine scharfe, eindeutig erkennbare Trennung der Erythrozyten, der weißen Blutzellen und des Plasmas abzeichnet, die mit einer bekannten Blutsenkung vergleichbar ist. Nach Eintritt der Interphase wurde die Spritze und der Edelstahlständer mit einem alkoholischen Desinfektionsmittel ohne Rückstandrest desinfiziert. Nach der Desinfektion wurde die Spritze aus dem Spritzenständer entnommen und unter eine Laminatflow gestellt, unter der sich bereits die weiteren benötigten Geräte, Verbrauchsmaterialien und Reagenzien befanden.

Dort erfolgte dann die weitere Präparation des sedimentierten Blutes in folgender Weise: An die Spritze wurde ein Butterfly-Schlauchsystem angebracht. Ferner wurden ein 50 ml -Blue Max-Röhrchen, ein 15 ml-Röhrchen und zwei Röhrchen mit je 10 ml Inhalt bereitgestellt. In das erste 10 ml-Röhrchen wurden 2 ml des Plasmas überführt, in dem der Zytokingehalt (=Zytokin-Ausgangswert) bestimmt wurde. Auch in das zweite 10 ml-Röhrchen wurden 10 ml Plasma eingeführt, in dem die infektionsserologischen Parameter anti-HCV, anti-HIV-1-2 und HBsAg bestimmt wurden. Anschließend wurde der Schlauch in das Blue Max-Röhrchen eingelegt, und es erfolgte die Überführung des Plasmas bis die Erythrozytenschicht (sichtbare rötliche Verfärbung des Schlauches) erreicht wurde. Unter Beibehaltung des Drucks auf den Spritzenstempel und weitere Überführung des Blutes in das Röhrchen nahm die Fließfähigkeit des Blutes ab. Der Druck auf den Spritzenstempel wurde solange fortgesetzt, bis die Fließfähigkeit wieder den Vorzustand (Einzeltropfenbildung) erreicht hat. Danach war die Überführung des Spritzeninhalts in das Röhrchen beendet.

Dann wurden 25 Vol.-% der Menge des im 50 ml-Blue Max-Röhrchens befindlichen Plasma/Zellgemisches (weiße Blutzellen) unter Benutzung einer sterilen serologischen Pipette (2 ml) mit Peleus-Ball aus diesem Röhrchen entnommen und in das 15 ml Röhrchen überführt. Dieses Röhrchen wurde in einer Tischzentrifuge 10 Minuten bei 1800 U/min zentrifugiert. Das Röhrchen wird aus der Zentrifuge entnommen und der Überstand in das 50 ml-Blue Max-Röhrchen dekantiert. Dann wurden folgende Aliquote in eine 250 ml-Zellkulturflasche eingebracht:
- in 10 ml Ringerlösung befindliches Zellgemisch nach Zentrifugation (nackte Zellen = demaskierte Zellen) aus dem 15 ml Röhrchen,
- unbehandelte Zellen (75 Vol.-% des Ausgangsgemisches) und Überstand nach Zentrifugation aus dem 50 ml-Blue Max-Röhrchen und
- Ringerphosphatlösung zum Auffüllen der Suspension auf 45 ml

Die Zellkulturflasche wurde dann liegend in einen Brutschrank gelegt und bei 37 ± 2 °C. inkubiert. Die Inkubationsdauer beträgt zwischen 43 und 77 Stunden. Nach Beendigung der Inkubationszeit wurde die Zellkulturflasche aus dem Brutschrank entnommen und senkrecht in einen Tiefkühlschrank gestellt und bei mindestens -16 °C während 12 Stunden bis 2 Wochen gelagert.

Das so erhaltene Produkt kann dann in üblicher Weise in Ampullen konfektioniert werden.

Auf diese Weise werden Produkte erhalten, die einen im Vergleich zum Ausgangswert erhöhten Zytokingehalt aufweisen.

Diese Produkte sind daher zur Behandlung von durch die Antigene verursachten Erkrankungen, insbesondere Tumorerkrankungen, bestens geeignet.

## Patentansprüche

1. Verfahren zur Herstellung von Humaneigenblutzytokinen, umfassend die folgenden Schritte:
a) Gewinnen einer Zusammensetzung von weißen Blutzellen, Plasma und einem Antigen aus dem Vollblut eines Spenders, wobei diese von den Erythrozyten abgetrennt werden,
b) Aufteilen der Zusammensetzung in ein erstes und ein zweites Aliquot,
c) Demaskieren der im ersten Aliquot vorliegenden Zellen und
d) Zusammengeben des in Schritt c) erhaltenen ersten Aliquots zum zweiten Aliquot und nachfolgende Inkubation.

2. Verfahren nach Anspruch 1, **dadurch gekennzeichnet, daß** das Antigen Tumorzellen sind.

3. Verfahren nach einem der Ansprüche 1 oder 2, **dadurch gekennzeichnet, daß** die Zytokine ausgewählt sind aus Tumornekrosefaktor-a, Interferon-g, und löslichem Interleukin-2-Rezeptor und Gemische von zwei oder allen dieser Interleukine.

4. Verfahren nach einem der Ansprüche 1 bis 3, **dadurch gekennzeichnet, daß** die Konzentration von mindestens zwei Zytokinen im Vergleich zu unbehandeltem Blut erhöht wird.

5. Verfahren nach einem der Ansprüche 1 bis 4, **dadurch gekennzeichnet, daß** die Zusammensetzung aus heparinisiertem Vollblut gewonnen wird.

6. Verfahren nach einem der Ansprüche 1 bis 5, **dadurch gekennzeichnet, daß** die weißen Blutzellen, das Plasma und die Antigene von den Erythrozyten durch Sedimentation abgetrennt werden.

7. Verfahren nach einem der Ansprüch 1 bis 6, **dadurch gekennzeichnet, daß** das Aufteilen in Schritt b) so erfolgt, daß das erste Aliquot 10 bis 40 Vol.-% des gesamten Volumens der Zusammensetzung beträgt.

8. Verfahren nach Anspruch 7, **dadurch gekennzeichnet, daß** das erste Aliquot 20 bis 30 Vol.-% beträgt.

9. Verfahren nach einem der Ansprüche 1 bis 8, **dadurch gekennzeichnet, daß** das Demaskieren in Schritt c) die Entfernung der Tarnung der Zellen umfaßt.

10. Verfahren nach einem der Ansprüche 1 bis 9, **dadurch gekennzeichnet, daß** das Demaskieren in Schritt c) die Membranreduktion der Zellen umfaßt.

11. Verfahren nach einem der Ansprüche 1 bis 10, **dadurch gekennzeichnet, daß** das Demaskieren der Zellen in Schritt c) spontan erfolgt oder induziert wird.

12. Verfahren nach Anspruch 11, **dadurch gekennzeichnet, daß** das Induzieren physikalisch erfolgt.

13. Verfahren nach Anspruch 12, **dadurch gekennzeichnet, daß** das physikalische Induzieren durch Zentrifugation erfolgt.

14. Verfahren nach einem der Ansprüche 1 bis 13, **dadurch gekennzeichnet, daß** die Inkubation bei 37 ± 2 °C während 48 ± 5 bis 72 ± 5 Stunden erfolgt.

15. Verfahren nach einem der Ansprüche 1 bis 14, **dadurch gekennzeichnet, daß** das nach Schritt d) erhaltene Produkt in Ampullen konfektioniert wird.

16. Verfahren nach einem der Ansprüche 1 bis 15, **dadurch gekennzeichnet, daß** das nach Schritt d) erhaltene Produkt bei ca. -16 °C oder weniger während 12 Stunden bis 14 Tagen gelagert wird.

## Claims

1. Method for the production of human blood cytokines, comprising the following steps:
a) Obtaining a composition of white blood cells, plasma and an antigen from the whole blood of a donor, wherein these are separated from the erythrocytes,
b) Dividing the composition into a first and a second aliquot,
c) Unmasking the cells present in the first aliquot and
d) Mixing together the first aliquot obtained in step c) with the second aliquot and subsequent incubation.

2. Method according to Claim 1, **characterized in that** the antigen are tumour cells.

3. Method according to any of Claims 1 or 2,
**characterized in that** the cytokines are selected from tumour necrosis factor-a, interferon-g, and soluble interleukin-2 receptor and mixtures of two or all of these interleukins.

4. Method according to any of Claims 1 to 3,
**characterized in that** the concentration of at least two cytokines is increased in comparison with untreated blood.

5. Method according to any of Claims 1 to 4,
**characterized in that** the composition is obtained from heparinsed whole blood.

6. Method according to any of Claims 1 to 5,
**characterized in that** the white blood cells, the plasma and the antigens are separated from the erythrocytes by sedimentation.

7. Method according to any of Claims 1 to 6,
**characterized in that** the division in step b) takes place such that the first aliquot is 10 to 40 vol. % of the total volume of the composition.

8. Method according to Claim 7, **characterized in that** the first aliquot is 20 to 30 vol. %.

9. Method according to any of Claims 1 to 8,
**characterized in that** the unmasking in step c) comprises the removal of the camouflage of the cells.

10. Method according to any of Claims 1 to 9,
**characterized in that** the unmasking in step c) comprises the membrane reduction of the cells.

11. Method according to any of Claims 1 to 10, **characterized in that** the unmasking of the cells in step c) takes place spontaneously or is induced.

12. Method according to Claim 11, **characterized in that** the induction takes place physically.

13. Method according to Claim 12, **characterized in that** the physical induction takes place by centrifugation.

14. Method according to any of Claims 1 to 13, **characterized in that** the incubation takes place at 37 ± 2 °C for 48 ± 5 to 72 ± 5 hours.

15. Method according to any of Claims 1 to 14, **characterized in that** the product obtained after step d) is assembled in ampoules.

16. Method according to any of Claims 1 to 15, **characterized in that** the product obtained after step d) is stored at approx. -16 °C or less for 12 hours to 14 days.

## Revendications

1. Procédé de fabrication de cytokines issues du sang de l'être humain concerné, comprenant les étapes suivantes :
a) Obtention, à partir du sang entier d'un donneur, d'une composition de globules blancs, de plasma et d'un antigène, ces composants étant séparés des érythrocytes.
b) Division de ladite composition en une première et une deuxième aliquote,
c) Démasquage des cellules présentes dans la première aliquote, et
d) Adjonction de la première aliquote obtenue dans l'étape c) à la deuxième aliquote, puis incubation.

2. Procédé selon la revendication 1, **caractérisé en ce que** l'antigène est constitué par des cellules tumorales.

3. Procédé selon l'une des revendications 1 ou 2, **caractérisé en ce que** les cytokines sont choisies parmi le facteur de nécrose tumorale-a, l'interféron-g et le récepteur soluble d'interleukine-2 et les mélanges de deux ou de toutes ces interleukines.

4. Procédé selon l'une des revendications 1 à 3, **caractérisé en ce que** la concentration d'au moins deux cytokines est augmentée par rapport à du sang non-traité.

5. Procédé selon l'une des revendications 1 à 4, **caractérisé en ce que** la composition est obtenue à partir de sang entier héparinisé.

6. Procédé selon l'une des revendications 1 à 5, **caractérisé en ce que** les globules blancs, le plasma et les antigènes sont séparés des érythrocytes par sédimentation.

7. Procédé selon l'une des revendications 1 à 6, **caractérisé en ce que** la division à l'étape b) est réalisée de façon à ce que la première aliquote représente 10 à 40 % en volume du volume total de la composition.

8. Procédé selon la revendication 7, **caractérisé en ce que** la première aliquote représente 20 à 30 % en volume.

9. Procédé selon l'une des revendications 1 à 8, **caractérisé en ce que** le démasquage à l'étape c) comprend la levée du camouflage des cellules.

10. Procédé selon l'une des revendications 1 à 9, **caractérisé en ce que** le démasquage à l'étape c) comprend la réduction membranaire des cellules.

11. Procédé selon l'une des revendications 1 à 10, **caractérisé en ce que** le démasquage des cellules à l'étape c) se produit spontanément ou est induit.

12. Procédé selon la revendication 11, **caractérisé en ce que** l'induction est réalisée de façon physique.

13. Procédé selon la revendication 12, **caractérisé en ce que** l'induction physique est réalisée par centrifugation.

14. Procédé selon l'une des revendications 1 à 13, **caractérisé en ce que** l'incubation est réalisée à 37 ± 2 °C durant 48 ± 5 à 72 ± 5 heures.

15. Procédé selon l'une des revendications 1 à 14, **caractérisé en ce que** le produit obtenu suite à l'étape d) est conditionné en ampoules.

16. Procédé selon l'une des revendications 1 à 15, **caractérisé en ce que** le produit obtenu suite à l'étape d) est stocké à environ. -16 °C ou moins durant 12 heures à 14 jours.
